# EUROPÄISCHE PATENTSCHRIFT

(11) **EP 2 585 121 B1**
(45) Veröffentlichungstag und Bekanntmachung des Hinweises auf die Patenterteilung: **26.03.2014**
(21) Anmeldenummer: 11725941.6
(22) Anmeldetag: 20.06.2011
(51) Int. Cl.: A61L 15/26, A61L 15/42, C08G 18/10, C08G 18/28, C08G 18/30, C08G 18/48, C08G 18/73, C08G 101/00

(54) **VERFAHREN ZUR HERSTELLUNG VON HYDROPHILEN, ALIPHATISCHEN POLYURETHAN-SCHÄUMEN MIT NIEDRIGER ROHDICHTE**
METHOD FOR PRODUCING HYDROPHILIC, ALIPHATIC POLYURETHANE FOAMS HAVING A LOW BULK DENSITY
PROCÉDÉ DE PRÉPARATION DE MOUSSES DE POLYURÉTHANE HYDROPHILES ALIPHATIQUES À FAIBLE DENSITÉ APPARENTE

(30) Priorität: 22.06.2010 EP 10166830
(43) Veröffentlichungstag der Anmeldung: 01.05.2013
(73) Patentinhaber: Bayer Intellectual Property GmbH, 40789 Monheim (DE)
(72) Erfinder: NIESTEN, Meike, 51061 Köln (DE); DÖRR, Sebastian, 40593 Düsseldorf (DE)
(74) Vertreter: BIP Patents
(86) Internationale Anmeldenummer: PCT/EP2011/060196
(87) Internationale Veröffentlichungsnummer: WO 2011/161041

(56) Entgegenhaltungen:
- EP-A1- 2 143 744
- EP-A1- 2 179 749
- WO-A1-00/47241

## Beschreibung

Die Erfindung betrifft ein Verfahren zur Herstellung von hydrophilen, aliphatischen Polyurethan-Schäumen mit niedriger Rohdichte. Weiterhin sind ein nach dem Verfahren erhältlicher hydrophiler, aliphatischer Polyurethan-Schaum und dessen Verwendung als Wundauflage, Inkontinenzprodukt oder als kosmetischer Artikel Gegenstände der Erfindung.

Aus der europäischen Patentanmeldung EP 2 143 744 A1 ist ein Verfahren bekannt, mit dessen Hilfe hydrophile, aliphatische Polyurethan-Schäume hergestellt werden können. Bei diesem Verfahren werden isocyanatfunktionelle Präpolymere mit C₈- bis C₂₂-Monocarbonsäuren oder deren Ammonium- oder Alkalisalzen oder C₁₂- bis C₄₄-Dicarbonsäuren oder deren Ammonium- oder Alkalisalzen und Wasser umgesetzt. Die Präpolymere sind durch Umsetzung von niedermolekularen, aliphatischen Diisocyanaten mit di- bis hexafunktionellen Polyalkylenoxiden erhältlich. Die Komponenten werden vermischt und in einen Becher gegeben, in dem dann die Schäumungsreaktion stattfindet. Dabei wird ein Polyurethan-Blockschaum erhalten. Dieser wird, wenn er beispielsweise als Wundauflage verwendet werden soll, auf die gewünschte Dicke von typischerweise 10 µ bis 5 cm zugeschnitten.

Bei dem Verfahren der EP 2 143 744 A1 werden ausschließlich Präpolymere eingesetzt, die einen Gehalt an niedermolekularen, aliphatischen Diisocyanaten von unter 1 Gewichtsprozent aufweisen. Auf diese Weise soll sicher gestellt werden, dass in dem fertigen Schaum keine extrahierbaren freien, niedermolekularen, aliphatischen Diisocyanate mehr enthalten sind, deren Freisetzung aus Wundauflagen als gesundheitlich bedenklich gilt. Um den gewünschten niedrigen Gehalt der Diisocyanate zu erhalten, ist bei dem Verfahren der EP 2 143 744 A1 die Entfernung überschüssiger niedermolekularer Diisocyanate nach der Präpolymerherstellung mittels Dünnschichten notwendig. Dies ist aufwendig.

Darüber hinaus war es wünschenswert, über ein Verfahren zu verfügen, mit dessen Hilfe Schäume hergestellt werden können, die gegenüber den nach dem bekannten Verfahren erhältlichen Schäumen eine niedrigere Rohdichte aufweisen. Eine niedrige Rohdichte ist vorteilhaft, da bei gleichem Volumen weniger Material notwendig ist und durch den hohen Anteil an Poren und die damit verbundene kapillare Wirkung ein schneller und guter Feuchtigkeitstransport möglich ist.

Aufgabe der vorliegenden Erfindung war daher ein einfaches Verfahren zur Herstellung von hydrophilen, aliphatischen Polyurethan-Schäumen mit niedriger Rohdichte und einem geringen Anteil an extrahierbaren freien Isocyanaten zu entwickeln.

Diese Aufgabe wird durch das Verfahren des Anspruchs 1 gelöst, bei dem
I) ein Gemisch aus isocyanatfunktionellen Präpolymeren A) und niedermolekularen, aliphatischen Diisocyanaten A1) durch Umsetzung von niedermolekularen, aliphatischen Diisocyanaten A1) einer Molmasse von 140 bis 278 g/mol mit
   di- bis hexafunktionellen Polyalkylenoxiden A2) einer OH-Zahl von 22,5 bis 112 mg KOH/g und einem Ethylenoxidanteil von 50 bis 100 mol%, bezogen auf die Gesamtmenge der enthaltenen Oxyalkylengruppen,
   wobei das Verhältnis der niedermolekularen, aliphatischen Diisocyanate A1) zu den dibis hexafunktionellen Polyalkylenoxiden A2) so eingestellt wird, das in dem resultierenden Gemisch A) der Gehalt an freiem niedermolekularen, aliphatischen Diisocyanaten A1) von 1 bis 15 Gew.-% beträgt,
   hergestellt wird,
II) das Gemisch des Schritts I) mit C8- bis C22-Monocarbonsäuren oder deren Ammonium- oder Alkalisalzen oder C12- bis C₄₄-Dicarbonsäuren oder deren Ammonium- oder Alkalisalzen B) und Wasser C) vermischt, und
III) das Gemisch des Schritts II) aufgeschäumt und ausgehärtet wird.

Überraschenderweise wurde gefunden, dass mit Hilfe des erfindungsgemäßen Verfahrens Polyurethan-Schäume erhalten werden können, die nicht nur eine im Vergleich zu Schäumen, die nach dem bekannten Verfahren hergestellten wurden, geringere Rohdichte haben, sondern darüber hinaus auch keinen höheren Anteil an extrahierbaren freien Verbindungen aufweisen. So hat sich herausgestellt, dass eine Entfernung des überschüssigen niedermolekularen, aliphatischen Diisocyanats nach der Präpolymerherstellung aus gesundheitlichen Gründen - im Bezug auf den Schaum -nicht notwendig ist und der vorhandene Überschuss an Diisocyanat sogar zu einer Verminderung der Rohdichte führt.

Gemäß einer bevorzugten Ausführungsform der Erfindung kann im Schritt I) das Verhältnis der niedermolekularen, aliphatischen Diisocyanate A1) zu den di- bis hexafunktionellen Polyalkylenoxiden A2) so eingestellt werden, dass in dem resultierenden Gemisch des Schritts I) der Gehalt an freiem, niedermolekularen, aliphatischen Diisocyanat A1) 1,5 bis 10, bevorzugt 2,0 bis 8 Gew.-% beträgt. In diesem Fall werden Schäume mit einer besonders niedrigen Rohdichte erhalten.

Die Umsetzung der Diisocyanate A1) mit den Polyalkylenoxiden A2) kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung kann typischerweise bei 25 bis 140°C, bevorzugt bei 60 bis 100°C erfolgen.

Beispiele für niedermolekulare, aliphatische Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, N orbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Hexamethylendiisocyanat, Isophorondiisocyanat, Butylendiisocyanat und ganz besonders bevorzugt sind Hexamethylendiisocyanat und Isophorondiisocyanat.

Bevorzugt ist auch, wenn als Diisocyanat A1) ausschließlich Hexamethylendiisocyanat und Isophorondiisocyanat oder deren Mischungen untereinander eingesetzt werden.

Die Polyalkylenoxide A2) sind bevorzugt Copolymere aus Ethylenoxid und Propylenoxid, die auf Polyolen oder Aminen gestartet sind und einem Ethylenoxidgehalt, bezogen auf die Gesamtmenge der enthaltenen Oxyallcylengruppen, von 50 bis 100 mol%, bevorzugt von 60 bis 85 mol%, aufweisen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide A2) besitzen typischerweise zahlenmittlere Molekulargewichte von 1000 bis 15000 g/mol, bevorzugt von 3000 bis 8500 g/mol.

Ferner können die Polyalkylenoxide A2) OH-Funktionalitäten von 2 bis 6, bevorzugt von 3 bis 6, besonders bevorzugt von 3 bis 4 besitzen.

In Weiterbildung der Erfindung ist vorgesehen, als Polyalkylenoxide A2) Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen, einzusetzen.

Vorzugsweise kann der NCO-Gehalt, bestimmt nach DIN-EN ISO 11909, des Gemischs I) aus isocyanatfunktionellen Präpolymeren A) und niedermolekularen, aliphatischen Diisocyanaten A1) nach der vollständigem Umsetzung der OH-Gruppen von A2) 4 bis 10 Gew.-% betragen. Die Verwendung eines derartigen Gemisches führt zu Schäumen mit einer besonders niedrigen Rohdichte.

Als Komponente B) werden Ammonium- und Alkalisalze von C₈- bis C₂₂-Monocarboxylaten oder deren freien Carbonsäuren oder Ammonium- und Alkalisalze von C₁₂- bis C₄₄-Dicarboxylaten oder deren freien Dicarbonsäuren, bevorzugt Kalium- oder Natriumsalze von C₈- bis C₂₂-Mono-carboxylaten oder von C₁₂- bis C₄₄-Dicarboxylaten und besonders bevorzugt Natriumsalze von C₈-bis C₂₂-Monocarboxylaten eingesetzt.

Beispiele geeigneter Verbindungen der Komponente B) sind die Ammonium-, Na-, Li- oder K-Salze von Ethylhexansäure, Octansäure, Decansäure, Dodecansäure, Palmitinsäure, Stearinsäure, den Octadecensäuren, den Octadecadiensäuren, den Octadecatriensäuren, Isostearinsäure, Erucasäure, Abietinsäure und ihren Hydrierungsprodukten. Beispiele für C₁₂- bis C₄₄-Dicarbonsäuren bzw. die daraus abgeleiteten Ammonium- und Alkalisalze sind Dodecandisäure, Dodecenyl-, Tetradecenyl-, Hexadecenyl- und Octadecenyl-Bernsteinsäure, C₃₆- und C₄₄-Dimerfettsäuren und ihre Hydrierungsprodukte sowie die entsprechenden Ammonium-, Na-, Li- oder K-Salze dieser Dicarbonsäuren.

Das einzusetzende Wasser C) kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel verwendet.

Es ist weiterhin möglich, dass das Gemisch des Schritts II) gegebenenfalls Katalysatoren D), Tenside E) Alkohole F) und / oder Treibmittel G) enthält.

Als Katalysatoren D) können insbesondere einzeln oder in Kombination Metallsalze, Amine, Amidine und Guanidine verwendet werden.

Zur Verbesserung der Schaumbildung, Schaumstabilität oder der Eigenschaften des resultierenden Polyurethan-Schaums können die Verbindungen der Komponente E) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder eingesetzt. Besonders bevorzugt werden EO/PO-Blockcopolymere eingesetzt. Bevorzugt werden allein die EO/PO-Blockcopolymere als Komponente E) eingesetzt.

Zudem können zur Verbesserung der Schaumeigenschaften des resultierenden Polyurethan-Schaums Verbindungen der Komponente F) eingesetzt werden. Hierbei handelt es sich grundsätzlich um alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Das Aufschäumen kann grundsätzlich durch das bei der Reaktion der Isocyanatgruppen mit Wasser gebildete Kohlendioxid erfolgen, die Verwendung von weiteren Treibmitteln G) ist jedoch ebenfalls möglich. So können prinzipiell auch Treibmittel aus der Klasse der Kohlenwasserstoffe wie C₃-C₆-Alkane, z.B. Butane, *n*-Pentan, *iso*-Pentan, *cyclo*-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Gemäß einer bevorzugten Ausführungsform der Erfindung ist vorgesehen, dass die Komponenten A) bis F) in den folgenden Mengen eingesetzt werden:
100 Gewichtsteile des Gemisches des Schritts I)
0,1 bis 5 Gewichtteile C8- bis C22-Monocarbonsäuren oder deren Ammonium- oder Alkalisalze oder C12- bis C₄₄-Dicarbonsäuren oder deren Ammonium- oder Alkalisalze B)
1 bis 200 Gewichtsteile Wasser C)
0 bis 1 Gewichtteile Katalysatoren D)
0 bis 10 Gewichtsteile Tenside E)
0 bis 20 Gewichtsteile Alkohole F)

Besonders bevorzugt werden die Komponenten A) bis F) in folgenden Mengen eingesetzt:
100 Gewichtsteile des Gemisches des Schritts I)
0,1 bis 5 Gewichtteile an C8- bis C22-Monocarbonsäuren oder deren Ammonium- oder Alkalisalze oder C12- bis C₄₄-Dicarbonsäuren oder deren Ammonium- oder Alkalisalze B)
2 bis 100 Gewichtsteile Wasser C)
0 bis 1 Gewichtteile an Katalysatoren D)
0 bis 10 Gewichtsteile Tenside E)
0 bis 20 Gewichtsteile Alkohole F).

Ganz besonders bevorzugt werden die Komponenten A) bis F) in folgenden Mengen eingesetzt:
100 Gewichtsteile des Gemisches des Schritts I)
0,1 bis 5 Gewichtteile an C8- bis C22-Monocarbonsäuren oder deren Ammonium- oder Alkalisalze oder C12- bis C₄₄-Dicarbonsäuren oder deren Ammonium- oder Alkalisalze B)
3 bis 50 Gewichtsteile Wasser C)
0 bis 1 Gewichtteile an Katalysatoren D)
0 bis 10 Gewichtsteile Tenside E)
0 bis 20 Gewichtsteile Alkohole F).

Beim Durchmischen der Komponenten und/oder Gemische und während der Schäumungsreaktion kann bei Temperaturen von 0 bis 100°C gearbeitet werden, bevorzugt bei 15 bis 70°C, ganz besonders bevorzugt bei 20 bis 50°C.

Nach dem Mischen der Komponente kann das Gemisch auf ein flächiges Substrat als Schicht mit konstanter Dicke aufgebracht werden. Geeignete Substrate sind zum Beispiel Trennfolien oder Trennpapiere, die perforiert sein können.

Vorzugsweise kann das Gemisch auf das Substrat aufgerakelt werden. Dazu kann das Gemisch in einem Rakelkasten gegossen und in einer bestimmten Dicke horizontal in flächigen Matten auf ein geeignetes Substrat wie z.B. eine Trennfolie oder einen Trennpapier gerakelt werden.

Die Spalthöhe des Rakels liegt dabei im Allgemeinen im Bereich von 0,2 bis 20 mm, bevorzugt von 0,2 bis 5 und ganz besonders bevorzugt von 0,2 bis 2 mm. Die Filmbreite des zu verwendenden Rakels kann dem jeweiligen Verwendungszweck angepasst werden. Beispiele sind Filmbreiten zwischen 10 und 5000 mm, bevorzug zwischen 10 und 4000 mm.

Als Rakel können alle bekannten Typen wie beispielsweise Luftrakel, Walzenrakel, Streichrakel, Kastenrakel, Messerrakel oder Magnetrakel eingesetzt werden. Als Material für die Rakel kommen alle üblichen Materialien in Betracht, z.B. Metalle wie Edelstahl oder Kunststoffe. Auch ein Verbund mehrerer Materialien zur Herstellung des Rakels ist möglich. Es können sowohl Handrakel als auch Maschinenrakel eingesetzt werden, bevorzugt ist die Verwendung von Maschinenrakeln, eingebunden in geeigneten Beschichtungsanlagen. Auch die Applikation zwischen Walzen ist möglich.

Auf die Schicht des Gemisches kann direkt nach dem Auftragen ein perforiertes Trennelement flächig aufgelegt, so dass es die dem Substrat abgewandte Oberfläche der Schicht bedeckt. Unter perforiert wird vorliegend ein Trennelement verstanden, dass eine Vielzahl von, das Trennelement von der Auflagefläche aus durchsetzenden Aussparungen aufweist. Die Aussparungen haben bevorzugt einen kreisförmigen Durchmesser. Bevorzugt ist auch, wenn die Aussparungen gleichmäßig über das Trennelement verteilt sind. Die Aussparungen können bevorzugt einen Durchmesser von 20 bis 300 µm haben. In diesem Fall erhält man Schäume, bei denen keine Erhebungen in Form der Aussparrungen des Trennelements auf der Schaumoberfläche sichtbar sind. Die Schäume haben eine glatte Oberfläche, was insbesondere bei deren Verwendung als Wundauflage vorteilhaft ist, da diese möglichst flächig am Körper anliegen sollen. Der Abstand zwischen zwei benachbarten Aussparungen liegt vorzugsweise zwischen 0,1 bis 5 mm, weiter bevorzugt zwischen 0,5 bis 3 mm und ganz besonders bevorzugt zwischen 0,8 und 2,5 mm. Bei dem perforierten Trennelement kann es sich beispielsweise um ein perforiertes Trennpapier oder eine perforierte Trennfolie handeln. Das Trennpapier kann z.B. silikonisiertes Papier, Polyolefin-beschichtetes Papier oder Fluorcarbon-beschichtetes Papier sein. Ebenso kann die Trennfolie aus Silkon, Polyolefinen und / oder Fluorcarbon bestehen, bzw. mit derartigen Materialien beschichtet sein. Bevorzugt kann nach dem Aufbringen das Trennelement insbesondere noch mit einem definierten Druck auf die Schicht aus dem Gemisch des Schritts II) gepresst werden.

Um die Aushärtung des Polyurethan-Schaums nach Beendigung der Expansion zu beschleunigen, kann dieser erwärmt werden. Vorzugsweise kann der Polyurethan-Schaum auf eine Temperatur von 40 und 140 °C, weiter bevorzugt von 60 bis 120 °C und besonders bevorzugt von 60 bis 110 °C erwärmt werden.

Besonders bevorzugt ist auch ein Verfahren, bei dem aus dem erfindungsgemäßen Polyurethan-Schaum eine Wundauflage hergestellt wird.

Weitere Gegenstände der Erfindung sind ein nach dem erfindungsgemäßen Verfahren herstellbarer Polyurethan-Schaum.

Die erhaltenen Polyurethan-Schäume weisen eine poröse, zumindest teilweise offenzellige Struktur mit miteinander kommunizierenden Zellen auf.

Die Polyurethan-Schäume können mit weiteren Materialien verklebt, laminiert oder beschichtet werden, beispielsweise auf Basis von Hydrogelen, (semi-) permeablen Folien, Schaumfolien, Beschichtungen, Hydrokolloiden oder anderen Schäumen.

Die erfindungsgemäßen Polyurethan-Schäume sind besonders geeignet zur Herstellung von Wundauflagen. Dabei können die Polyurethan-Schäume in direktem oder indirektem Kontakt mit der Wunde sein. Bevorzugt werden die Polyurethan-Schäume jedoch in direktem Kontakt mit der Wunde eingesetzt, um beispielsweise eine optimale Absorption von Wundflüssigkeit zu gewährleisten. Die Polyurethan-Schäume zeigen keine Zelltoxizität (Bestimmung nach ISO 10993-5 und ISO 10993-12).

Die Polyurethan-Schäume, welche als Wundauflage eingesetzt werden, können zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung, beispielsweise durch Gammastrahlung, erfolgt. Die Bestrahlung kann dabei gegebenenfalls unter Schutzgasatmosphäre erfolgen. Die erfindungsgemäßen Polyurethan-Schäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Gegenstand der Erfindung ist schließlich auch ein erfindungsgemäßer hydrophiler, aliphatischer Polyurethan-Schaum zur Verwendung als Wundauflage, Inkontinenzprodukt oder kosmetischer Artikel.

### Beispiele

Sofern nicht abweichend gekennzeichnet, beziehen sich alle Prozentangaben auf das Gewicht. Die Bestimmung der Festkörpergehalte erfolgte nach DIN-EN ISO 3251. Die Bestimmung der Viskositäten erfolgte bei 23 °C und wurde nach DIN 53019 durchgeführt. Die NCO-Gehalte wurden volumetrisch gemäß DIN-EN ISO 11909 bestimmt.

### Bestimmung der Extraktmenge:

48 h: 10 g des Schaums wurden für 48 Stunden in 300 ml vollentsalztes Wasser bei 37°C eingelegt und durch Titration des chemischen Sauerstoffbedarfs gemäß DIN EN 1484 die Extraktmenge bestimmt.

7 Tage: 4,7 g des Schaums wurden für 7 Tage in 220 ml vollentsalztes Wasser bei 37°C eingelegt und durch Titration des chemischen Sauerstoffbedarfs gemäß DIN EN 1484 die Extraktmenge bestimmt.

### Bestimmung der Rohdichte

Zur Bestimmung der Rohdichte wurde zunächst ein 10x10x5 cm großes Stück des jeweiligen Schaums gewogen. Anschließend wurde die Rohdichte berechnet, indem die Masse des Schaums durch sein Volumen geteilt wurde.

### Verwendete Substanzen und Abkürzungen:

- Carboxylat 1:: 5 % Natriumoleat in Wasser
- Desmodur^{®} N 3400:: Aliphatisches Polyisocyanat (HDI-Uretdion), NCO-Gehalt 21,8 %

### Beispiel 1: Herstellung des Polyurethan-Präpolymers 1, Dünnschichtverfahren, für Vergleichsversuch

Zu einem Gemisch aus 1000g Hexamethylendiisocyanat (HDI) und 1g Benzoylchlorid wurde bei 80°C innerhalb von 3h 1000g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 2h bei einem Druck von 10 mbar getrocknet wurde, zugetropft und für 12h nachgerührt. Das überschüssige HDI wurde durch Dünnschichtdestillation bei 130°C und 0,1 mbar entfernt, wobei die nicht flüchtigen Bestandteile mit 1g Chlorpropionsäure stabilisiert wurden. Man erhielt ein Präpolymer mit einem NCO-Gehalt von 2,8 % und einer Viskosität von 3500 mPas.

### Beispiel 2: Herstellung des Polyurethan-Präpolymers 2

Zu einem Gemisch aus 504g HDI und 1g Benzoylchlorid wurde bei 80°C innerhalb von 2h 1781g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 2h bei einem Druck von 10mbar getrocknet wurde, zugetropft und für 12h bei 80°C nachgerührt. Man erhielt ein Präpolymer gemischt mit freiem HDI mit einem NCO-Gehalt von 8,7 % und einer Viskosität von 2700 mPas.

### Beispiel 3: Herstellung des Polyurethan-Präpolymers 3

Zu einem Gemisch aus 437g HDI und 2,4g Benzoylchlorid wurde bei 80°C innerhalb von 2h 1929g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 2h bei einem Druck von 10mbar getrocknet wurde, zugetropft und für 12h bei 80°C nachgerührt. Man erhielt ein Präpolymer gemischt mit freiem HDI mit einem NCO-Gehalt von 6,7 % und einer Viskosität von 5500 mPas.

### Beispiel 4: Herstellung des Polyurethan-Pränolymers 4

Zu einem Gemisch aus 328g HDI und 2,5g Benzoylchlorid wurde bei 80°C innerhalb von 2h 1929g eines Polyalkylenoxids mit einer Molmasse von 4680 g/mol gestartet auf Glycerin, einem Ethylenoxidgewichtsanteil von 72 % und Propylenoxidgewichtsanteil von 28 %, das zuvor bei 100°C während 2h bei einem Druck von 10mbar getrocknet wurde, zugetropft und für 12h bei 80°C nachgerührt. Man erhielt ein Präpolymer gemischt mit freiem HDI mit einem NCO-Gehalt von 4,7 % und einer Viskosität von 18500 mPas.

### Beispiele 5 bis 7, Vergleichbeispiele 1 und 2: Herstellung von Schäumen

Die Isocyanat-Komponenten wurden 15 Sekunden mit einer Rührerdrehzahl von 1200 Upm homogenisiert. Dann wurden die weiteren Komponenten eingewogen und weitere 10 Sekunden verrührt. Schließlich wurde die Mischung in eine Papierform von 15x15x15 (Breite x Höhe x Länge) gegossen. Als Oligomer wurde, falls verwendet, dabei jeweils Desmodur® N 3400 eingesetzt; als Carboxylat eine 5 Gew.-%ige Lösung von Natriumoleat in Wasser. Darüber hinaus zugesetztes Wasser ist extra angegeben.

| | **Beispiel 5** | **Beispiel 6** | **Beispiel 7** | **Vergleichsbeispiel 1** | **Vergleichsbeispiel 2** |
|---|---|---|---|---|---|
| Präpolymer 2 | 75 | | | | |
| Präpolymer 3 | | 75 | | | |
| Präpolymer 4 | | | 75 | | |
| Prepolymer 1 (DünnschichtVerfahren) | | | | 75 | 75 |
| Oligomer (Desmodur N3400) | | | | 8,33 | |
| NCO-Gehalt Mischung [%] | 8,7 | 6,7 | 4,7 | 4,6 | 2,6 |
| Gehalt monomeres HDI [Gew.-%] | 14,4 | 10,3 | 6,4 | 0 | 0 |
| Wasser | 3,35 | 3,35 | 3,35 | 3,35 | 3,35 |
| DBU | 0,05 | 0,05 | 0,05 | 0,05 | 0,05 |
| Carboxylat 1 | 8,15 | 8,15 | 8,15 | 8,15 | 8,15 |
| Rohdichte [g/1000 cm³] | 69,5 | 67 | 90 | 109 | 199 |
| Extrakt nach EN 1484 [Gew.-%] | | | | | Nicht bestimmt |
| 48 Stunden | 0,12 | 0,11 | 0,14 | 0,11 | |
| 7 Tage | 0,14 | 0,12 | 0,16 | 0,15 | |

Die Beispiele 5 bis 7 zeigen, dass auf Basis von Präpolymeren, die freies niedermolekulares, aliphatisches Diisocyanat enthalten, Schäume mit einer Rohdichte von kleiner als 100 g/l hergestellt werden können. Die Extraktmenge dieser Schäume ist dabei nicht größer als die des Schaums aus dem Vergleichsbeispiel 1, der auf einem gedünnschichteten Präpolymer basiert, dass frei von monomerem Diisocyanat ist.

## Patentansprüche

1. Verfahren zur Herstellung von hydrophilen, aliphatischen Polyurethan-Schäumen, bei dem
I) ein Gemisch aus isocyanatfunktionellen Präpolymeren A) und niedermolekularen, aliphatischen Diisocyanaten A1) durch Umsetzung von
niedermolekularen, aliphatischen Diisocyanaten A1) einer Molmasse von 140 bis 278 g/mol mit
di- bis hexafunktionellen Polyalkylenoxiden A2) einer OH-Zahl von 22,5 bis 112 mg KOH/g und einem Ethylenoxidanteil von 50 bis 100 mol%, bezogen auf die Gesamtmenge der enthaltenen Oxyalkylengruppen,
wobei das Verhältnis der niedermolekularen, aliphatischen Diisocyanate A1) zu den dibis hexafunktionellen Polyalkylenoxiden A2) so eingestellt wird, das in dem resultierenden Gemisch A) der Gehalt an freiem niedermolekularen, aliphatischen Diisocyanaten A1) von 1 bis 15 Gew.-% beträgt,
hergestellt wird,
II) das Gemisch des Schritts I) mit C8- bis C22-Monocarbonsäuren oder deren Ammonium- oder Alkalisalzen oder C12- bis C₄₄-Dicarbonsäuren oder deren Ammonium- oder Alkalisalzen B) und Wasser C) vermischt, und
III) das Gemisch des Schritts II) aufgeschäumt und ausgehärtet wird.

2. Verfahren nach Anspruch 1, **dadurch gekennzeichnet, dass** im Schritt I) das Verhältnis der niedermolekularen, aliphatischen Diisocyanate A1) zu den di- bis hexafunktionellen Polyalkylenoxiden A2) so eingestellt wird, dass in dem resultierenden Gemisch des Schritts I) der Gehalt an freiem, niedermolekularen, aliphatischen Diisocyanat A1) 1,5 bis 10, bevorzugt 2,0 bis 8 Gew.- % beträgt.

3. Verfahren nach einem der Ansprüche 1 oder 2, **dadurch gekennzeichnet, dass** der NCO-Gehalt, bestimmt nach DIN-EN ISO 11909, des Gemischs aus isocyanatfunktionellen Präpolymeren A) und niedermolekularen, aliphatischen Diisocyanaten A1) nach vollständiger Umsetzung der OH-Gruppen von A2) 4 bis 10 Gew.-% beträgt.

4. Verfahren nach einem der Ansprüche 1 bis 3, **dadurch gekennzeichnet, dass** als Diisocyanat A1) ausschließlich Hexamethylendiisocyanat, Isophorondiisocyanat oder deren Mischungen untereinander eingesetzt werden.

5. Verfahren nach einem der Ansprüche 1 bis 4, **dadurch gekennzeichnet, dass** als Polyalkylenoxide A2) Copolymere aus Ethylenoxid und Propylenoxid mit einem Ethylenoxidgehalt, bezogen auf die Gesamtmenge der enthaltenen Oxyalkylengruppen, von 60 bis 85 mol%, gestartet auf Polyolen oder Aminen eingesetzt werden.

6. Verfahren nach einem der Ansprüche 1 bis 5, **dadurch gekennzeichnet, dass** die Polyalkylenoxide A2) zahlenmittlere Molekulargewichte von 3000 bis 8500 g/mol aufweisen.

7. Verfahren nach einem der Ansprüche 1 bis 6, **dadurch gekennzeichnet, dass** die Polyalkylenoxide A2) OH-Funktionalitäten von 3 bis 4 aufweisen.

8. Verfahren nach einem der Ansprüche 1 bis 7, **dadurch gekennzeichnet, dass** das Gemisch des Schritts II) gegebenenfalls Katalysatoren D), Tenside E), Alkohole F) und / oder Treibmittel G) enthält.

9. Verfahren nach Anspruch 8 **dadurch gekennzeichnet, dass** die Komponenten A) bis F) in den folgenden Mengen eingesetzt werden:
100 Gewichtsteile des Gemisches des Schritts I)
0,1 bis 5 Gewichtteile C8- bis C22-Monocarbonsäuren oder deren Ammonium- oder Alkalisalze oder C12- bis C₄₄-Dicarbonsäuren oder deren Ammonium- oder Alkalisalze B)
1 bis 200 Gewichtsteile Wasser C)
0 bis 1 Gewichtteile Katalysatoren D)
0 bis 10 Gewichtsteile Tenside E)
0 bis 20 Gewichtsteile Alkohole F).

10. Verfahren nach einem der Ansprüche 1 bis 9, **dadurch gekennzeichnet, dass** der Polyurethan-Schaum nach Beendigung der Aufschäumung zur Beschleunigung der Aushärtung erwärmt wird, bevorzugt auf eine Temperatur von 40 und 140 °C, weiter bevorzugt von 60 bis 120 °C und besonders bevorzugt von 60 bis 110 °C.

11. Verfahren nach einem der Ansprüche 1 bis 10, **dadurch gekennzeichnet, dass** aus dem Polyurethan-Schaum eine Wundauflage hergestellt wird.

12. Hydrophiler, aliphatischer Polyurethan-Schaum, erhältlich nach einem Verfahren gemäß einem der Ansprüche 1 bis 11.

13. Hydrophiler, aliphatischer Polyurethan-Schaum nach Anspruch 12 zur Verwendung als Wundauflage. Inkontinenzprodukt oder kosmetischer Artikel.

14. Verwendung eines hydrophilen, aliphatischen Polyurethan-Schaums nach Anspruch 12 zur Herstellung eines Mittels zur Behandlung von Wunden.

## Claims

1. Method for producing hydrophilic aliphatic polyurethane foams which comprises
I) preparing a mixture of isocyanate-functional prepolymers A) and low molecular weight aliphatic diisocyanates A1) by reaction of
low molecular weight aliphatic diisocyanates A1) having a molar mass of 140 to 278 g/mol with
di- to hexafunctional polyalkylene oxides A2) having an OH number of 22.5 to 112 mg KOH/g and an ethylene oxide fraction of 50 to 100 mol%, based on the total amount of oxyalkylene groups present,
the ratio of the low molecular weight aliphatic diisocyanates A1) to the di- to hexafunctional polyalkylene oxides A2) being set such that the free low molecular weight aliphatic diisocyanate A1) content in the resulting mixture A) is 1 to 15 wt.%,
II) mixing the mixture of step I) with C8-to C22-monocarboxylic acids or their ammonium or alkali metal salts or C12- to C44-dicarboxylic acids or their ammonium or alkali metal salts B) and water C), and
III) foaming and curing the mixture of step II).

2. Method according to Claim 1, **characterized in that** in step I) the ratio of the low molecular weight aliphatic diisocyanates A1) to the di- to hexafunctional polyalkylene oxides A2) is set such that the free low molecular weight aliphatic diisocyanate A1) content in the resulting mixture of step I) is 1.5 to 10, preferably 2.0 to 8 wt%.

3. Method according to either Claim 1 or 2, **characterized in that** the NCO content, determined to DIN-EN ISO 11909, of the mixture of isocyanate-functional prepolymers A) and low molecular weight aliphatic diisocyanates A1) after complete reaction of the OH groups of A2) is 4 to 10 wt%.

4. Method according to any of Claims 1 to 3, **characterized in that** the diisocyanate A1) used is exclusively hexamethylene diisocyanate, isophorone diisocyanate or mixtures thereof.

5. Method according to any of Claims 1 to 4, **characterized in that** the polyalkylene oxides A2) used are copolymers of ethylene oxide and propylene oxide having an ethylene oxide content, based on the total amount of oxyalkylene groups present, of 60 to 85 mol% and started on polyols or amines.

6. Method according to any of Claims 1 to 5, **characterized in that** the polyalkylene oxides A2) have number-average molecular weights of 3000 to 8500 g/mol.

7. Method according to any of Claims 1 to 6, **characterized in that** the polyalkylene oxides A2) have OH functionalities of 3 to 4.

8. Method according to any of Claims 1 to 7, **characterized in that** the mixture of step II) optionally contains catalysts D), surfactants E), alcohols F) and/or blowing agents G).

9. Method according to Claim 8, **characterized in that** the components A) to F) are used in the following amounts:
100 parts by weight of the mixture of step I),
0.1 to 5 parts by weight of C8- to C22-monocarboxylic acids or their ammonium or alkali metal salts or C12- to C44-dicarboxylic acids or their ammonium or alkali metal salts B),
1 to 200 parts by weight of water C),
0 to 1 part by weight of catalysts D),
0 to 10 parts by weight of surfactants E),
0 to 20 parts by weight of alcohols F).

10. Method according to any of Claims 1 to 9, **characterized in that** the polyurethane foam on completion of foaming is heated, preferably to a temperature of 40 to 140°C, more preferably of 60 to 120°C and even more preferably of 60 to 110°C, to speed curing.

11. Method according to any of Claims 1 to 10, **characterized in that** a wound dressing is produced from the polyurethane foam.

12. Hydrophilic aliphatic polyurethane foam obtainable according to a method according to any of Claims 1 to 11.

13. Hydrophilic aliphatic polyurethane foam according to Claim 12 for use as wound dressing, incontinence product or cosmetic article.

14. Use of a hydrophilic aliphatic polyurethane foam according to Claim 12 for preparing a composition for treating wounds.

## Revendications

1. Procédé pour la préparation de mousses de polyuréthanes aliphatiques, hydrophiles, dans lequel
I) on prépare un mélange de prépolymères à fonctionnalité isocyanate A) et de diisocyanates aliphatiques de bas poids moléculaire A1) par transformation de
diisocyanates aliphatiques de bas poids moléculaire A1), présentant une masse molaire de 140 à 278 g/mole avec
des poly(oxydes d'alkylène) difonctionnels à hexafonctionnels A2) présentant un indice d'OH de 22,5 à 112 mg de KOH/g et une proportion d'oxyde d'éthylène de 50 à 100% en mole par rapport à la quantité totale des groupes d'oxyalkylène contenus,
le rapport des diisocyanates aliphatiques de bas poids moléculaire A1) aux poly(oxydes d'alkylène) difonctionnels à hexafonctionnels A2) étant réglé de manière telle que dans le mélange résultant A), la teneur en diisocyanates aliphatiques de bas poids moléculaire libres A1) est de 1 à 15% en poids,
II) on mélange le mélange de l'étape I) avec des acides C₈-C₂₂-monocarboxyliques ou leurs sels d'ammonium ou de métal alcalin ou des acides C₁₂-C₄₄-dicarboxyliques ou leurs sels d'ammonium ou de métal alcalin B) et de l'eau C), et
III) le mélange de l'étape II) est moussé et durci.

2. Procédé selon la revendication 1, **caractérisé en ce que** dans l'étape I), le rapport des diisocyanates aliphatiques de bas poids moléculaire A1) aux poly(oxydes d'alkylène) difonctionnels à hexafonctionnels A2) est réglé de manière telle que dans le mélange résultant de l'étape I), la teneur en diisocyanate aliphatique de bas poids moléculaire libre A1) est de 1,5 à 10% en poids, de préférence de 2,0 à 8% en poids.

3. Procédé selon l'une quelconque des revendications 1 ou 2, **caractérisé en ce que** la teneur en NCO, déterminée selon la norme DIN-EN ISO 11909, du mélange de prépolymères à fonctionnalité isocyanate A) et de diisocyanates aliphatiques de bas poids moléculaire A1) après la transformation complète des groupes OH de A2) vaut 4 à 10% en poids.

4. Procédé selon l'une quelconque des revendications 1 à 3, **caractérisé en ce qu'**on utilise, comme diisocyanate A1), exclusivement du diisocyanate d'hexaméthylène, du diisocyanate d'isophorone ou leurs mélanges les uns avec les autres.

5. Procédé selon l'une quelconque des revendications 1 à 4, **caractérisé en ce qu'**on utilise comme poly(oxydes d'alkylène) A2) des copolymères d'oxyde d'éthylène et d'oxyde de propylène présentant une teneur en oxyde d'éthylène, par rapport à la quantité totale des groupes d'oxyalkylène contenus, de 60 à 85% en mole, initiés sur des polyols ou des amines.

6. Procédé selon l'une quelconque des revendications 1 à 5, **caractérisé en ce que** les poly(oxydes d'alkylène) A2) présentent des poids moléculaires numériques moyens de 3000 à 8500 g/mole.

7. Procédé selon l'une quelconque des revendications 1 à 6, **caractérisé en ce que** les poly(oxydes d'alkylène) A2) présentent des fonctionnalités OH de 3 à 4.

8. Procédé selon l'une quelconque des revendications 1 à 7, **caractérisé en ce que** le mélange de l'étape II) contient le cas échéant des catalyseurs D), des tensioactifs E), des alcools F) et/ou des agents gonflants G).

9. Procédé selon la revendication 8, **caractérisé en ce que** les composants A) à F) sont utilisés dans les quantités suivantes :
100 parties en poids du mélange de l'étape I)
0, 1 à 5 parties en poids d'acides C₈-C₂₂-monocarboxyliques ou leurs sels d'ammonium ou de métal alcalin ou d'acides C₁₂-C₄₄-dicarboxyliques ou leurs sels d'ammonium ou de métal alcalin B),
1 à 200 parties en poids d'eau C),
0 à 1 partie en poids de catalyseurs D),
0 à 10 parties en poids de tensioactifs E),
0 à 20 parties en poids d'alcools F).

10. Procédé selon l'une quelconque des revendications 1 à 9, **caractérisé en ce que** la mousse de polyuréthane est chauffée, après la fin du moussage, pour accélérer le durcissement, de préférence à une température de 40 à 140°C, plus préférablement de 60 à 120°C et de manière particulièrement préférée de 60 à 110°C.

11. Procédé selon l'une quelconque des revendications 1 à 10, **caractérisé en ce qu'**un pansement est fabriqué à partir de la mousse de polyuréthane.

12. Mousse de polyuréthane aliphatique, hydrophile, pouvant être obtenue selon un procédé selon l'une quelconque des revendications 1 à 11.

13. Mousse de polyuréthane aliphatique, hydrophile selon la revendication 12 destinée à une utilisation comme pansement, produit d'incontinence ou article cosmétique.

14. Utilisation d'une mousse de polyuréthane aliphatique, hydrophile selon la revendication 12 pour la préparation d'un agent destiné au traitement de plaies.
